# EUROPÄISCHE PATENTSCHRIFT

(11) **EP 3 879 976 B1**
(45) Veröffentlichungstag und Bekanntmachung des Hinweises auf die Patenterteilung: **08.01.2025**
(21) Anmeldenummer: 19805118.7
(22) Anmeldetag: 13.11.2019
(51) Int. Cl.: A01K 11/00, A01K 29/00

(54) **VERFAHREN, VORRICHTUNG UND SYSTEM ZUR ERMITTLUNG ZUMINDEST EINER ZUSTANDSGRÖSSE DES ORGANISMUS EINES NUTZTIERES**
METHOD, DEVICE, AND SYSTEM FOR ASCERTAINING AT LEAST ONE STATUS PARAMETER OF A LIVESTOCK ORGANISM
PROCÉDÉ, APPAREIL ET SYSTÈME POUR DÉTERMINER AU MOINS UN PARAMÈTRE D'ÉTAT DE L'ORGANISME D'UN ANIMAL D'ÉLEVAGE

(30) Priorität: 13.11.2018 AT 509862018
(43) Veröffentlichungstag der Anmeldung: 22.09.2021
(73) Patentinhaber: smaXtec animal care GmbH, 8010 Graz (AT)
(72) Erfinder: ASTL, Michael, 8020 Graz (AT); KULICH, Philipp, 8010 Graz (AT); BRANDSTÄTTER, Stefan, 8010 Graz (AT); FRECH, Manuel, 4573 Hinterstoder (AT); WUTTE, Matthias, 8042 Graz (AT); ROSENKRANZ, Stefan, 8010 Graz (AT)
(74) Vertreter: Patentanwaltskanzlei Matschnig & Forsthuber OG
(86) Internationale Anmeldenummer: PCT/AT2019/060386
(87) Internationale Veröffentlichungsnummer: WO 2020/097655

(56) Entgegenhaltungen:
- WO-A1-2017/103239
- DE-A1- 19 901 124
- US-A1- 2009 187 392
- US-A1- 2018 271 066
- US-A1- 2018 310 885
- US-B2- 10 098 328

## Beschreibung

Die Erfindung betrifft ein Verfahren zur Ermittlung zumindest einer Zustandsgröße des Organismus zumindest eines Nutztieres, wobei zumindest eine Sondenvorrichtung zur Messung zumindest eines physikalischen Parameters im Magen-Darmtrakt des Nutztieres und zumindest eine Auswerteeinheit außerhalb des Magen-Darmtraktes des Nutztieres angeordnet ist. Die Erfindung betrifft außerdem eine Sondenvorrichtung zur Messung zumindest einer Zustandsgröße des Organismus eines Nutztieres, wobei die Sondenvorrichtung im Magen-Darmtrakt des Nutztieres anordenbar ist. Darüber hinaus betrifft die Erfindung ein System mit zumindest einer Auswerteeinheit und zumindest einer derartigen Sondenvorrichtung.

Die Landwirtschaft entwickelt sich weltweit zunehmend in Richtung Großbetriebe. Dabei wird beispielsweise für Landwirte in der Nutztierhaltung das Herdenmanagement, vor allem hinsichtlich der gesundheitlichen Einzeltierüberwachung oder der leistungsgerechten Futterzuteilung, immer schwieriger. Die Parameter, die für das Wohlbefinden und den Futterbedarf der einzelnen Tiere eine Rolle spielen, umfassen neben äußeren Zustandsinformationen wie beispielsweise Temperatur, Sauerstoffgehalt oder Luftfeuchtigkeit in der Luft im Stall auch physiologische Parameter wie beispielsweise pH-Wert des Magensaftes, Körpertemperatur oder andere.

Mit ansteigender Betriebsgröße ist aufgrund dieser Vielzahl von Parametern eine individualisierte Futtergabe kaum mehr möglich; auch können Krankheitssymptome bei einzelnen Tieren häufig nicht rechtzeitig erkannt werden. Um dennoch eine artgerechte Haltung und ein ökonomisches Produzieren zu ermöglichen, ist es für den Landwirt von enormer Bedeutung, über die Parameter, die für das Wohlbefinden der Tiere eine Rolle spielen, Bescheid zu wissen.

Dazu wird vermehrt auf elektronische Managementhilfen zurückgegriffen. Beispielsweise beschreibt die DE 199 01 124 A1 eine bolusförmige Sonde, die Sensoren zur Messung verschiedener physiologischer Zustandsgrößen wie Druck, Temperatur, Leitfähigkeit, pH-Wert oder Ammoniakgehalt messen kann und in den Magen-Darmtrakt eines Rinds eingebracht wird. Die AT 509 255 B1 der Anmelderin beschreibt eine ähnliche Sondeneinheit, bei der die Messdaten drahtlos übertragen werden können, wobei die Messsensorik zum Schutz vor mechanischer Beschädigung innerhalb eines säurebeständigen Gehäuses zumindest teilweise von einer zylindrischen Schutzvorrichtung umgeben ist. Die Sondeneinheit kann dabei Daten an in einem Stall verteilte Basisstationen funken und über diese von einer Steuervorrichtung bedient bzw. ausgelesen werden.

Bei derartigen Vorrichtungen ist insbesondere zu berücksichtigen, dass eine langfristige Funktion entsprechend der Lebens- bzw. Nutzungsdauer des Nutztieres benötigt wird und gleichzeitig das Auslesen der Daten durch das Anordnen innerhalb des Nutztieres eine gewisse Sendeleistung erfordert.

Daher wird zunehmend auch auf rein physikalische Messmethoden gesetzt, die eine Verlängerung der Laufzeiten derartiger Vorrichtungen versprechen.

Beispielsweise beschreibt die WO 2017/103239 A1 eine Vorrichtung, bei der insbesondere Magendruck, -temperatur und -motilität eines Nutztieres erfasst werden. Dazu wird ein Drucksensor verwendet, der durch einen gasdichten Bereich mit elastisch verformbarer Wandung die Bewegungen des Magens, also die Magenmotilität, erfassen und über zumindest 60 Tage Messwerte gewinnen kann. Darüber hinaus ist die Verwendung von Temperatur- und Bewegungssensoren zur Ermittlung der Gesamtbewegung des Tieres beschrieben, wobei die ermittelten Daten über eine telemetrische Einrichtung an einen Empfänger geschickt werden können.

Nachteilig daran ist insbesondere, dass Datenaufnahme und -versand mit großem Energieaufwand erfolgen und eine Verwendung nur über einen begrenzten Zeitraum möglich ist.

Auch die DE 299 11 803 U1 beschreibt eine in den Magen eines Nutztieres einbringbare Vorrichtung zur Bestimmung der Magenmotilität, also der Stärke und Frequenz von Magenbewegungen. Dazu ist ein piezoelektrischer Sensor vorgesehen, der die Druckänderungen im Magen des Nutztieres registriert und dessen Daten nach Rückgewinnung über eine Computerschnittstelle ausgelesen oder während des Betriebs an eine Empfangsstation übertragen werden. Im Gegensatz zu Beschleunigungssensoren soll so die *"Motilität, bzw. deren Wirkung auf die Nahrung im Magen",* direkt gemessen werden. Nachteilig daran sind insbesondere die zeitlich begrenzte Verwendbarkeit und die Ungenauigkeit der Messung, die sich durch die verwendete Technologie ergibt.

Die WO 2016/036303 A1 schlägt eine Lösung vor, bei der ein Beschleunigungssensor am Nacken eines Nutztieres befestigt ist, um Kaubewegungen des Kiefers beim Wiederkäuen zu messen und damit Wiederkäuzeiten zu bestimmen. Da der Sensor nicht innerhalb des Nutztieres angeordnet ist kann so eine gute Datenübertragung sichergestellt werden, außerdem kann eine Energieversorgungseinheit unkompliziert gewechselt werden. Nachteilig ist allerdings insbesondere, dass durch die externe Anordnung am Nacken Bewegungssignale mitgemessen werden, die keine direkte Aussage über die Gesundheit des Nutztieres erlauben. Des Weiteren kann es zu Beschädigungen kommen, wenn das Nutztier mit dem Beschleunigungssensor an Hindernisse oder andere Tiere stößt oder diesen abzustreifen versucht. US2018271066 A1 offenbart eine Lösung, bei der ein Beschleunigungssensor im Magen-Darmtrakt eines Nutztieres angeordnet ist.

Es ist daher eine Aufgabe der Erfindung, die oben genannten Nachteile zu beheben und ein Verfahren bzw. eine Sondenvorrichtung und ein diese beinhaltendes System bereitzustellen, mit dem bzw. der auf einfache und zuverlässige Weise über einen langen Zeitraum gesundheitsrelevante Zustandsgrößen von Nutztieren erhoben werden können. Diese Aufgabe wird durch die Merkmale von Anspruch 1 gelöst. Vorteilhafterweise handelt es sich bei der ersten Zustandsgröße um eine Zustandsgröße, die in Schritt c) mit geringerer Datenübertragungsrate übermittelbar ist als der erste physikalische Parameter und/oder die zweite Zustandsgröße. Damit wird durch die Umwandlung die benötigte Bandbreite und Übertragungsdauer reduziert, was zu einem reduzierten Stromverbrauch auf Seiten der Sondenvorrichtung und geringstmöglicher Belastung des Übertragungsmediums führt, was insbesondere bei realer Verwendung unzähliger Sondenvorrichtungen in landwirtschaftlichen Betrieben einen wichtigen Faktor darstellt. In einer Variante der Erfindung kann die erste Zustandsgröße vor der Übertragung in Schritt c) einem Kompressionsverfahren unterzogen werden, um die zu übertragende Datenrate weiter zu reduzieren und so die Vorteile hinsichtlich Stromverbrauch und Bandbreite zu erhöhen.

Günstigerweise ist der erste physikalische Parameter mit geringerem Rechenaufwand in die erste Zustandsgröße umwandelbar als der erste physikalische Parameter in die zweite Zustandsgröße und/oder die erste Zustandsgröße in die zweite Zustandsgröße. Dadurch können die ressourcen- und damit energieaufwändigen Vorgänge auf die Auswerteeinheit verschoben werden, während die Sondenvorrichtung diesbezüglich geschont wird.

Erfindungsgemäß erfolgt die Ermittlung des ersten physikalischen Parameters in Schritt a) auf zumindest eine der folgenden Arten: kontinuierlich; in regelmäßigen Abständen; kontinuierlich, sobald ein von der Sondenvorrichtung gemessener physikalischer Parameter, insbesondere der erste physikalische Parameter, einen Schwellwert überschritten hat; in regelmäßigen Abständen, sobald ein von der Sondenvorrichtung gemessener physikalischer Parameter, insbesondere der erste physikalische Parameter, einen Schwellwert überschritten hat; kontinuierlich oder in regelmäßigen Abständen für einen vordefinierten Zeitraum, sobald ein von der Sondenvorrichtung gemessener physikalischer Parameter, insbesondere der erste physikalische Parameter, einen Schwellwert überschreitet. Erfindungsgemäß wird der Schwellwert entweder vordefiniert auf der Sondenvorrichtung hinterlegt oder von der Auswerteeinheit anlassbezogen an die Sondenvorrichtung übermittelt oder besteht aus einem Basis-Schwellwert und einem in Abhängigkeit vom Verhalten des ersten physikalischen Parameters anpassbaren, zum Basis-Schwellwert addierten oder vom Basis-Schwellwert subtrahierten Dynamik-Wert. Damit kann zusätzlich Energie der Sondenvorrichtung gespart werden.

Erfindungsgemäß handelt es sich bei dem ersten physikalischen Parameter in Schritt a) um die Beschleunigung in alle drei Raumrichtungen eines kartesischen Koordinatensystems.

Bei der ersten Zustandsgröße in Schritt b) handelt es sich günstigerweise um die Motilität, insbesondere um die Dauer und/oder die Periodizität und/oder die Frequenz der Motilität. Aus der Motilität lassen sich vielfältige Informationen über den Zustand eines Nutztieres gewinnen.

Um die Messwerte in einen Gesamtkontext zu stellen bzw. um zeitliche Präzision zu ermöglichen wird in Schritt b) die erste Zustandsgröße mit einem Echtzeitsignal eines in der Sondenvorrichtung vorgesehenen Taktgebers und/oder einem Temperatursignal eines in der Sondenvorrichtung vorgesehenen Temperatursensors verifiziert.

Ein hoher praktischer Nutzen des Verfahrens ergibt sich dann, wenn es sich bei der zweiten Zustandsgröße in Schritt d) um eine der folgenden Zustandsgrößen des Organismus des Nutztieres handelt: Rumination, Herzschlag, Fresszeit (kann sowohl Beginn und/oder Ende als auch Dauer des Fressens beinhalten). Mit anderen Worten können als zweite Zustandsgrößen insbesondere den Zustand und Fortschritt des Verdauungstraktes/-vorgangs betreffende Größen herangezogen werden, beispielsweise die Rumination bzw. Wiederkäuaktivität anhand der Dauer der Kontraktionen der Motilität und/oder deren Periodizität und/oder Frequenz.

In einer Variante des Verfahrens ist Schritt a) in folgende Unterschritte unterteilt:
a1) Ermitteln der Beschleunigung der Sondenvorrichtung in einer oder mehreren Raumrichtungen eines kartesischen Koordinatensystems;
a2) Ermitteln des ersten physikalischen Parameters durch Durchführen zumindest eines der folgenden Schritte: Summieren von Absolutwerten der in Schritt a1) ermittelten Beschleunigungswerte, Summieren der Quadrate der in Schritt a1) ermittelten Beschleunigungswerte, Wurzel der Summe der Quadrate der in Schritt a1) ermittelten Beschleunigungswerte, arctan der in Schritt a1) ermittelten Beschleunigungswerte für y-Achse und z-Achse des kartesischen Koordinatensystems, arctan der Wurzel der Summe der Quadrate der in Schritt a1) ermittelten Beschleunigungswerte und der x-Achse des kartesischen Koordinatensystems.

Erfindungsgemäß wird in Schritt c) die erste Zustandsgröße als binäres Rechtecksignal an die Auswerteeinheit übermittelt. Erfindungsgemäß wird in Schritt b) zur Ermittlung der ersten Zustandsgröße Messwerten des ersten physikalischen Parameters, die größer oder gleich einem Schwellwert sind, der Wert *"1"* zugeordnet und Messwerten des ersten physikalischen Parameters, die kleiner sind als ein Schwellwert, der Wert *"0"*. Dadurch lässt sich die Datenmenge, die zu übertragen ist, drastisch reduzieren.

Günstigerweise wird unmittelbar vor Schritt c) in einer Sondensteuereinheit der Sondenvorrichtung und/oder vor Durchführung des Schritts d) in der Auswerteeinheit ein Validierungsschritt durchgeführt, um die Plausibilität der Werte der ersten Zustandsgröße des Organismus des Nutztieres zu prüfen. Mit anderen Worten wird entweder in der Sondensteuereinheit der Sondenvorrichtung, in der Auswerteeinheit oder in beiden mit einem Validierungsschritt geprüft, ob die Werte der ersten Zustandsgröße des Organismus plausibel sind. Plausibel bedeutet hier, dass die Werte innerhalb der zulässigen bzw. erwarteten bzw. in der Anwendung auftretenden Bereiche liegen, wie sie insbesondere in der Sondensteuereinheit oder der Auswerteeinheit oder beiden hinterlegt werden. Für plausible Werte erfolgt die Durchführung von Schritt c), also das Übermitteln an die Auswerteeinheit und/oder auf der Auswerteeinheit das Umwandeln in die zweite Zustandsgröße. Nicht plausible Werte werden für das weitere Verfahren nicht herangezogen, also nicht oder ersetzt durch einen Fehlercode an die Auswerteeinheit übermittelt und/oder nicht für die Auswertung in der Auswerteeinheit herangezogen. Damit kann verhindert werden, dass Fehlmessungen, die z.B. durch harsche Umweltbedingungen verursacht sein können, die Genauigkeit der Ergebnisse nachteilig beeinflussen.

Die Aufgabe der Erfindung wird weiters durch eine eingangs erwähnte Sondenvorrichtung erfindungsgemäß dadurch gelöst, dass die Sondenvorrichtung im Magen-Darmtrakt des Nutztieres anordenbar ist und zumindest folgende, in einem Gehäuse angeordnete Komponenten aufweist:
- zumindest ein Sensorelement zur Messung zumindest eines physikalischen Parameters im Magen-Darmtrakt des Nutztieres,
- zumindest eine Sendeeinrichtung, vorzugsweise mit zumindest einer Antenne, zum drahtlosen Übertragen und Empfangen von Information, und
- zumindest eine Sondensteuereinheit die zur Durchführung der Schritte a) bis c) eines oben genannten Verfahrens eingerichtet ist.

Günstigerweise ist in der Sondenvorrichtung zumindest eines der folgenden Sensorelemente vorgesehen: Beschleunigungssensor, Beschleunigungssensor zur Messung der Beschleunigung in alle drei Raumrichtungen eines kartesischen Koordinatensystems, Temperatursensor, pH-Sensor, Taktgeber, Echtzeituhr, Kameraelement. Auch andere Sensortypen können verwendet werden.

Mehr Möglichkeiten zur Verwendung der erfindungsgemäßen Sondenvorrichtung ergeben sich dann, wenn zumindest ein mit der Sondensteuereinheit verbundenes Speicherelement vorgesehen ist, insbesondere zumindest ein RAM- und/oder zumindest ein ROM-Speicherelement. Dadurch können physikalische Parameter bzw. Zustandsgrößen gespeichert werden, falls keine unmittelbare Übertragung an eine Auswerteeinheit möglich oder gewünscht ist.

Die Aufgabe der Erfindung wird außerdem durch ein eingangs erwähntes System mit zumindest einer Auswerteeinheit und zumindest einer oben beschriebenen Sondenvorrichtung gelöst, wobei mit dem System das oben beschriebene Verfahren durchführbar ist. Dadurch wird eine umfassende Überwachung von größeren Nutztierherden ermöglicht.

Die Erfindung wird nachfolgend anhand eines nicht einschränkenden Ausführungsbeispiels, das in den Zeichnungen dargestellt ist, näher erläutert. Die Zeichnungen dienen lediglich Illustrationszwecken und schränken somit die Erfindung in keiner Weise ein. Darin zeigen
Fig. 1 eine Kuh als beispielhaftes Nutztier und die Anordnung einer Sondeneinheit in deren Magen-Darmtrakt;
Fig. 2 eine Seitenansicht einer erfindungsgemäßen Sondenvorrichtung im geschlossenen Zustand;
Fig. 3 eine schematische Darstellung einer erfindungsgemäßen Sondenvorrichtung und deren Komponenten;
Fig. 4 eine schematische Darstellung eines erfindungsgemäßen Systems mit mehreren Sondenvorrichtungen und einer Auswerteeinheit;
Fig. 5 ein Ablaufdiagramm einer ersten Variante des erfindungsgemäßen Verfahrens;
Fig. 6 Messergebnisse eines ersten physikalischen Parameters aus dem Verfahrensablauf in Fig. 5;
Fig. 7 Ergebnisse für eine erste Zustandsgröße des Organismus eines Nutztieres aus dem Verfahrensablauf in Fig. 5;
Fig. 8 ein Ablaufdiagramm einer zweiten Variante des erfindungsgemäßen Verfahrens;
Fig. 9 Messergebnisse eines ersten physikalischen Parameters aus dem Verfahrensablauf in Fig. 8; und
Fig. 10 Ergebnisse für eine erste Zustandsgröße des Organismus eines Nutztieres aus dem Verfahrensablauf in Fig. 8.

Gleiche Elemente sind in den verschiedenen Figuren aus Gründen der Übersichtlichkeit mit demselben Bezugszeichen versehen.

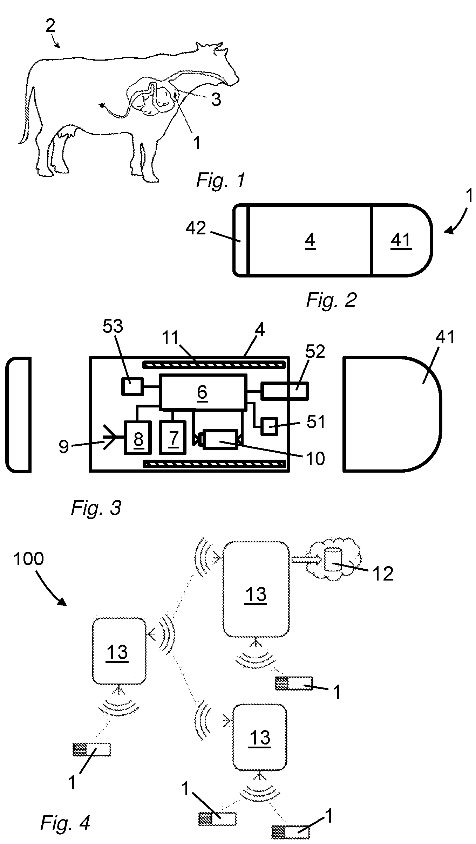

Fig. 1 zeigt das Schnittbild einer Kuh 2, wobei die Kuh 2 hier nur als mögliches Beispiel für ein Nutztier, insbesondere ein wiederkäuendes Nutztier genannt wird, in dessen Magen-Darmtrakt 3 eine Sondenvorrichtung 1 gemäß dem Ausführungsbeispiel der Erfindung eingebracht werden kann. Andere geeignete wiederkäuende Nutztiere wären beispielsweise Schafe, Ziegen oder auch Wildwiederkäuer wie Rotwild.

Das von der Kuh 2 aufgenommene und zerkaute Futter gelangt in deren Magen-Darmtrakt 3, beispielsweise in den Pansen oder den Netzmagen (*"Reticulum"*)*.* Aus dem Netzmagen wird das aufgenommene Futter einerseits in den Pansen weitertransportiert, andererseits zum Wiederkäuen in das Maul der Kuh 2 rücktransportiert. Aus der Messung von Zustandsgrößen des Organismus der Kuh 2 bzw. des Inhalts des Magen-Darmtrakts 3 lassen sich eventuelle Auswirkungen bzw. Rückschlüsse auf den Gesundheitszustand des Tieres ermitteln. Bei einem zu geringen pH-Wert kann es beispielsweise zu einer gefährlichen Pansenacidose kommen, Veränderungen von Herzschlagrate, Pansenmotilität, Wiederkäu- und Bewegungsaktivität lassen z.B. Rückschlüsse auf das Vorliegen von Milchfieber zu. Die Sondenvorrichtung 1 wird daher im Magen-Darmtrakt 3 des Tieres angeordnet, um über die Ermittlung physikalischer Parameter Zustandsgrößen des Organismus des Nutzieres bestimmbar zu machen. Insbesondere lassen sich gute Ergebnisse erzielen, wenn sich die Sondenvorrichtung 1 dauerhaft in einer Endposition im Netzmagen befindet.

Fig. 2 zeigt schematisch eine Seitenansicht der Sondenvorrichtung 1, während in Fig. 3 eine schematische, teiltransparente Ansicht eines Ausführungsbeispiels der Sondenvorrichtung 1 dargestellt ist: Innerhalb eines Gehäuses 4 mit einem ersten Verschlusselement 41 und einem zweiten Verschlusselement 42 sind ein erstes 51 und ein zweites Sensorelement 52 angeordnet. Bei dem ersten Sensorelement 51 kann es sich um einen Beschleunigungssensor handeln, das zweite Sensorelement 52 kann als Temperatursensor ausgeführt sein. Zusätzlich bzw. stattdessen können auch andere Sensoren verwendet werden, z.B. solche zur Messung von Temperatur, pH-Wert, Dichte, Druck, Leitfähigkeit, Schall, optischen Eigenschaften oder von Sauerstoff, CO₂, Ammoniak, Glukose, flüchtigen Fettsäuren, Acetat, Propionat, Butyrat und Laktat. Des Weiteren ist in der Sondenvorrichtung 1 ein Taktgeber 53, z.B. eine RTC (*"Real Time Clock"*) vorgesehen. Der Taktgeber 53 kann wie beschrieben als RTC ausgeführt sein und einen absoluten Zeitwert ausgeben. In einer Variante kann der Taktgeber 53 auch einen relativen Zeitwert ausgeben, z.B. die vergangene Zeit bzw. Taktanzahl seit Aktivieren der Sensorvorrichtung 1. Das Ermitteln eines absoluten Zeitwerts kann dann nachgelagert erfolgen, z.B. auf einer Auswerteeinheit 12 (siehe Fig. 4 und zugehörige Beschreibung weiter unten).

Die Sensorelemente 51, 52 und der Taktgeber 53 stehen mit einer Sondensteuereinheit 6 in Verbindung, die zur Steuerung der Sondenvorrichtung 1 dient. Die Sondensteuereinheit 6 ist beispielsweise als entsprechend programmierter Mikroprozessor ausgeführt. Die Sondensteuereinheit 6 kontrolliert und verarbeitet die Daten von den Sensorelementen 51, 52. Zur Speicherung der Daten kann ein Speicherelement 7 vorgesehen sein, beispielsweise ein Speicherchip oder eine SD-Karte. Das Speicherelement 7 speichert sowohl Messwerte der Sensorelemente 51, 52 als auch Betriebsparameter der Sondenvorrichtung 1 wie Funkfrequenz (zur Kommunikation mit einer Auswerteeinheit 12), Sendekanal, Systemzeit, aber auch Konfigurationsparameter wie z.B. die Abtastrate des Beschleunigungssensors, Abtastrate der Umwandlung in die erste Zustandsgröße und andere.

Über eine Sendeeinrichtung 8, die über eine Antenne 9 verfügt, werden Daten übertragen, beispielsweise an eine Auswerteeinheit 12, die in der Umgebung des Nutztieres 2 angeordnet ist (siehe Fig. 4). Günstigerweise ist die Sendeeinrichtung 8 als Sende-Empfangseinrichtung ausgeführt, die Daten sowohl senden als auch empfangen kann.

Die Energieversorgung der Sondenvorrichtung 1 erfolgt z.B. über eine Energieversorgungsvorrichtung 10, die beispielsweise als Batterie, Akkumulator oder Kondensator (vorteilhafterweise ein Dünnschicht- oder Superkondensator) ausgeführt sein kann. Auch ein Wiederaufladen durch *"Energy-Harvesting"* oder andere Methoden ist möglich.

Im dargestellten Ausführungsbeispiel sind die beschriebenen Komponenten gemäß der eingangs erwähnten AT 509 255 B1 innerhalb des Gehäuses 4 von einer hohlen, zumindest die Energieversorgungsvorrichtung 10 umgebenden Schutzvorrichtung 11 umschlossen, die vor mechanischer Einwirkung schützt. Damit wird verhindert, dass das Nutztier die Sondenvorrichtung 1 und insbesondere die darin angeordneten Komponenten, speziell die Energieversorgungsvorrichtung 10, zerbeißt, wenn sie im Zuge des Wiederkäuens ins Maul zurückgewürgt wird. Die Schutzvorrichtung 11 kann dabei aus einem beliebigen, widerstandsfähigen Material gefertigt sein, beispielsweise aus Kunststoffen oder Metallen.

Fig. 4 zeigt ein Ausführungsbeispiel eines Systems 100 mit mehreren beschriebenen Sondenvorrichtungen 1 - aus Gründen der Übersichtlichkeit sind die Nutztiere, in deren Magen-Darmtrakt die Sondenvorrichtungen 1 angeordnet sind, nicht dargestellt - und einer Auswerteeinheit 12, mit der die Sondenvorrichtungen 1 drahtlos kommunizieren. Zur Erhöhung der Reichweite, bzw. um die notwendige Sendeleistung zu reduzieren, sind im dargestellten Ausführungsbeispiel zwischen Auswerteeinheit 12 und den Sondenvorrichtungen 1 mehrere Sende-/Empfangseinheiten 13 vorgesehen, die als Relais fungieren, aber nicht zwingend vorhanden sein müssen. Dadurch lässt sich eine sternförmige Architektur zur Anwendung des LoRa-Netzwerkprotokolls (bzw. "*LoRaWAN" - "Long Range Wide Area Network"*) umsetzen, wobei die Sondenvorrichtungen 1 die Endgeräte darstellen und die Sende-/Empfangseinheiten 13 als Gateways, die Datenpakete an die Auswerteeinheit 12, z.B. einen Netzwerkserver, senden. Die Auswerteeinheit 12 kann also beispielsweise ein mobiler bzw. stationärer Computer sein, auf dem die entsprechenden Auswerteroutinen ablaufen, bzw. kann über das Internet eine Verbindung zu einem entsprechenden Server bestehen.

Bei den beschriebenen Sondenvorrichtungen 1 wird insbesondere darauf geachtet, einen möglichst lange dauernden Betrieb zu ermöglichen. Dies kann einerseits durch die Verwendung leistungsstarker Energieversorgungsvorrichtungen 10 erzielt werden, andererseits durch besonders sparsamen Betrieb. Dieser lässt sich vorteilhafterweise realisieren, indem in der Sondenvorrichtung 1 bzw. deren Sondensteuereinheit 6 möglichst nur wenig rechenintensive Vorgänge ablaufen, die komplexen Berechnungen aber in die Auswerteeinheit 12 verlagert werden, die außerhalb des Magen-Darmtraktes 3 des Nutztieres angeordnet ist und daher leichter z.B. unter Heranziehung des Stromnetzes betrieben werden kann. Zusätzlich wird darauf geachtet, die Datenmenge, die von der Sondenvorrichtung 1 zur Auswerteeinheit 12 übertragen wird, bei maximalem Informationsinhalt möglichst gering zu halten.

Im beschriebenen Verfahren wird daher in einem Schritt a) ein erster physikalischer Parameter durch die Sondenvorrichtung 1, bzw. deren Sensorvorrichtungen 51, 52, ermittelt. Als physikalische Parameter werden hierbei Parameter verstanden, die die physischen Bedingungen im Magen-Darmtrakt 3 des Nutztieres kennzeichnen, z.B. Temperatur, pH-Wert, Beschleunigungswerte, Bildinformationen und dergleichen. Im vorliegenden Ausführungsbeispiel wird die Beschleunigung, insbesondere die Beschleunigung in alle drei Raumrichtungen eines kartesischen Koordinatensystems, in dem sich die Sondenvorrichtung 1 befindet, herangezogen, die mit dem ersten Sensorelement 51, dem Beschleunigungssensor, ermittelt wird.

Zur besonders energiesparenden Ermittlung des ersten physikalischen Parameters erfolgt diese auf zumindest eine der folgenden Arten:
- Kontinuierlich, die Sondenvorrichtung 1 ist also immer aktiviert;
- in regelmäßigen Abständen, die Sondenvorrichtung 1 wird also zu bestimmten Zeitpunkten für die Messung aktiviert, bleibt dazwischen aber deaktiviert oder zumindest teilweise in einem energiesparenden Zustand;
- kontinuierlich, sobald ein von der Sondenvorrichtung 1 gemessener physikalischer Parameter, insbesondere der erste physikalische Parameter, einen Schwellwert überschritten hat;
- in regelmäßigen Abständen, sobald ein von der Sondenvorrichtung 1 gemessener physikalischer Parameter, insbesondere der erste physikalische Parameter, einen Schwellwert überschritten hat;
- kontinuierlich oder in regelmäßigen Abständen für einen vordefinierten Zeitraum, sobald ein von der Sondenvorrichtung 1 gemessener physikalischer Parameter, insbesondere der erste physikalische Parameter, einen Schwellwert überschreitet.

Der besagte Schwellwert wird entweder vordefiniert auf der Sondenvorrichtung 1 hinterlegt oder von der Auswerteeinheit 12 anlassbezogen an die Sondenvorrichtung 1 übermittelt oder besteht aus einem Basis-Schwellwert und einem in Abhängigkeit vom Verhalten des ersten physikalischen Parameters anpassbaren, zum Basis-Schwellwert addierten oder vom Basis-Schwellwert subtrahierten Dynamik-Wert. Dieser Dynamik-Wert kann beispielsweise als Mittelwert von Messergebnissen des ersten physikalischen Parameters über einen gewissen Zeitraum berechnet werden. Der Basis-Schwellwert kann auch identisch Null gesetzt werden, so dass der Schwellwert durch den Dynamik-Wert gebildet wird.

Die Beschleunigung als erster physikalischer Parameter bzw. die Messwerte des ersten physikalischen Parameters werden in einem Schritt b) in der Sondensteuereinheit 6 in eine erste Zustandsgröße des Organismus des Nutztieres 2 umgewandelt. Neben dem schwellwertabhängigen Betrieb der Sondenvorrichtung 1 kann auch die Sondensteuereinheit 6 selektiv betrieben werden. Sie wird beispielsweise aktiviert, wenn eine bestimmte Anzahl an Messwerten (z.B. 32 Messwerte) zumindest einer der Sensorvorrichtungen 51, 52 vorliegt und eine Verständigung per Interrupt an die Sondensteuereinheit 6 erfolgt, die beispielsweise als Microcontroller ausgeführt ist. Weitere Gründe für das Aktivieren der Sondensteuereinheit 6 können beispielsweise das Erreichen definierter Messintervalle (Messung Temperatur, pH-Wert, etc.) oder vorgegebene Sendeintervalle sein, wo ein Übertragungsversuch der gemessenen Daten gestartet wird. Derartige Intervalle werden mittels Timer/Counter (z.B. dem als RTC ausgeführten Taktgeber 53) umgesetzt, die Sondensteuereinheit 6 wird hier ebenfalls per Interrupts aktiviert.

Mit anderen Worten befindet sich in einer Variante der Erfindung die Sondensteuereinheit 6 standardmäßig in einem Ruhemodus und das Aktivieren der Sondensteuereinheit 6 aus dem Ruhemodus und das darauf folgende Umwandeln des ersten physikalischen Parameters in eine erste Zustandsgröße in der Sondensteuereinheit 6 (siehe Schritt b) weiter oben) erfolgt in Abhängigkeit vom Vorliegen einer Mindestanzahl von Messwerten und/oder vom Erreichen vorgegebener Messwerte. Unter einem Ruhemodus wird hier ein vollständiges Deaktivieren oder ein Energiesparmodus der Sondensteuereinheit 6 verstanden.

Zusätzlich oder stattdessen kann sich die Sondensteuereinheit 6 auch hinsichtlich des Übermittelns der ersten Zustandsgröße an die Auswerteeinheit 12 (Schritt c) weiter oben) in einem Ruhemodus befinden, bis ein gewisses vorgegebenes Sendeintervall erreicht ist, also ein Zeitpunkt, zu dem eine Übermittlung der Daten erfolgt.

Als erste Zustandsgröße des Organismus des Nutztieres 2 wird im beschriebenen Ausführungsbeispiel die Motilität, also die Pansen- bzw. Magenaktivität, herangezogen. Bei der Motilität handelt es sich im Wesentlichen um intestinale Bewegungen des Magen-Darmtraktes 3 des Nutztieres 2, die sich durch entsprechende Kontraktionen zeigt. Mit anderen Worten bezeichnet Motilität im Rahmen der vorliegenden Offenbarung Kontraktionen des Magen-Darmtraktes 3, die in Form von Beschleunigungskräften auf die Sondenvorrichtung 1 einwirken. Die Motilität als erste Zustandsgröße äußert sich daher als Beschleunigungskräfte, die aufgrund der Bewegungen des Magen-Darmtraktes 3, die sich unter anderem durch den Mageninhalt fortpflanzen, durch den Beschleunigungssensor der Sondenvorrichtung 1 messbar sind. Als erste Zustandsgröße kann die Motilität direkt, aber auch die Dauer der Kontraktionen der Motilität und/oder deren Periodizität bzw. Frequenz herangezogen werden.

Aus den Beschleunigungsdaten innerhalb des Magen-Darmtraktes 3 der Kuh 2 wird also die Motilität ermittelt. Dabei können Störsignale wie z.B. die Erdbeschleunigung aus den Beschleunigungsdaten herausgefiltert werden, beispielsweise durch Gleichanteilunterdrückung. Ohne Herausfiltern der Erdbeschleunigung ließe sich beispielsweise auch Lageinformation ermitteln, also Steh- und Liegezeiten des Nutztieres.

Durch Verwendung eines Taktgebers 53 bzw. der RTC wird das Signal der ersten Zustandsgröße, hier also das Motilitätssignal, zeitcodiert bzw. mit Echtzeit abgeglichen. Üblicherweise ist dabei auf der RTC die UTC *("universal coordinated time"*) gespeichert. In einer Variante der Erfindung ist die RTC bzw. der Taktgeber 53 in die Sondensteuereinheit 6 integriert, diese ist also als Microcontroller mit intergrierter RTC ausgeführt. In einer weiteren Variante kann absolute Zeitinformation von der Auswerteeinheit 12 an die Sondenvorrichtung 1 übermittelt werden, die diese mit einer eigenen relativen Zeitinformation (Anzahl von Takten oder *"Ticks"*) kombiniert. Wie weiter oben bereits beschrieben kann auch der Taktgeber 53 der Sensoreinheit 1 relative Zeitwerte ermitteln, die auf der Auswerteeinheit 12 in absolute Zeitwerte umgewandelt werden.

Zusätzlich kann das Signal eines Temperatursensors 52 herangezogen werden um Ausreißer des Signals der ersten Zustandsgröße zu identifizieren, die sich z.B. ergeben, wenn das Nutztier trinkt: Wenn die Motilität als erste Zustandsgröße verwendet wird könnten ja die Bewegungen im Magen-Darmtrakt 3, die sich durch das beim Trinken aufgenommene Medium ergeben, als Kontraktionen fehlinterpretiert werden. Durch die Berücksichtigung des Temperatursignals kann das verhindert werden. Dieses Temperatursignal kann dabei auch in der Auswerteeinheit 12 berücksichtigt werden. Dazu kann zusätzlich zur Übermittlung der ersten Zustandsgröße Information zu Temperatur, Zeit und sonstigen Statusdetails an die Auswerteeinheit 12 übermittelt werden.

In einem Schritt c) wird die erste Zustandsgröße, bzw. die Werte der Zustandsgröße, an die Auswerteeinheit 12 übermittelt. Insbesondere kommt hier eine drahtlose Übermittlung per Funk mit einem entsprechend passenden Protokoll (z.B. LoRa, ZigBee, RFiD, WLAN, oder andere) zum Einsatz, wobei vorzugsweise ein geeigneter Frequenzbereich, z.B. 300 MHz bis 450 MHz, verwendet wird, bei dem die Permeabilität für Radiowellen bei Tieren besonders hoch ist.

Diese Übermittlung kann kontinuierlich geschehen, real ist aber eine Übermittlung nicht immer möglich oder gewünscht, weil sich die Kuh 2 nicht im Empfangsbereich der Auswerteeinheit 12 befindet oder weil aus Energiespargründen die Daten nur paketweise verschickt werden sollen. Aus diesem Grund ist das als RAM und/oder ROM ausgeführte Speicherelement 7 vorgesehen. Beispielsweise können die Werte der ersten Zustandsgröße während eines Messintervalls im Direktzugriffsspeicher (*"RAM"*) gespeichert werden, wobei sie danach vom temporären Speicher in einen EEPROM, also einen Nur-Lesespeicher, übertragen werden. Der Zwischenschritt mit dem EEPROM ist insbesondere günstig, um ausreichende Datenmengen speichern zu können, bevor die nächste Übertragung zur Auswerteeinheit 12 möglich ist.

Erfindungsgemäß handelt es sich bei der ersten Zustandsgröße um eine Zustandsgröße, die mit einer geringeren Datenübertragungsrate übermittelbar ist als der erste physikalische Parameter und/oder eine zweite Zustandsgröße. Dadurch ergibt sich der Vorteil, dass das Übermitteln der Daten an die Auswerteeinheit 12 mit geringstmöglichem Stromverbrauch und geringstmöglicher Bandbreite erfolgen kann, was die Lebensdauer der Sondenvorrichtung 1 bzw. deren Energieversorgungsvorrichtung 10 drastisch erhöht.

Zusätzlich kann die erste Zustandsgröße vor der Übertragung einem Kompressionsverfahren unterzogen werden, um die zu übertragende Datenmenge weiter zu reduzieren. Dazu sind verschiedene Verfahren möglich, beispielsweise Median-Filterung zur Glättung des Signals, Entprellung oder ein Plausibilitätscheck (Übertragung einer 1er-Folge bzw. eines Fehlercodes, wenn die Messwerte nicht in einem gewissen Bereich liegen).

In einem Schritt d), der in der Auswerteeinheit 12 vorgenommen wird, erfolgt ein Umwandeln der ersten Zustandsgröße in eine zweite Zustandsgröße. Mit anderen Worten werden aus den Werten der ersten Zustandsgröße die Werte der zweiten Zustandsgröße ermittelt. Bei der zweiten Zustandsgröße handelt es sich z.B. um die Rumination, den Herzschlag, die Fress- und Trinkzeiten bzw. den Zustand und Fortschritt des Verdauungstraktes/-vorgangs betreffende Größen, beispielsweise auch die Dauer der Kontraktionen der Motilität und/oder deren Periodizität bzw. Frequenz, insoweit diese nicht als erste Zustandsgröße bereits auf der Sondeneinheit 1 ermittelt wurden. Zusätzlich können als zweite Zustandsgröße weitere Verhaltensinformationen in Form von Aktivitäten wie Stehen, langsames/schnelles Bewegen und Liegen übertragen werden.

Rumination bezeichnet im Rahmen der vorliegenden Offenbarung die Wiederkäuaktivität des Nutztieres, insbesondere deren Intensität, Dauer und Periodizität bzw. Frequenz.

Durch die Wahl von Beschleunigung als erstem physikalischen Parameter bzw. Motilität und Rumination als Zustandsgrößen lässt sich ein geringer Stromverbrauch auf der Sondenvorrichtung 1 realisieren, da der erste physikalische Parameter mit geringerem Rechenaufwand in die erste Zustandsgröße umwandelbar ist als der erste physikalische Parameter in die zweite Zustandsgröße und/oder die erste Zustandsgröße in die zweite Zustandsgröße. Die rechenintensiven, energieaufwändigen Umwandlungen finden also auf der Auswerteeinheit 12 statt.

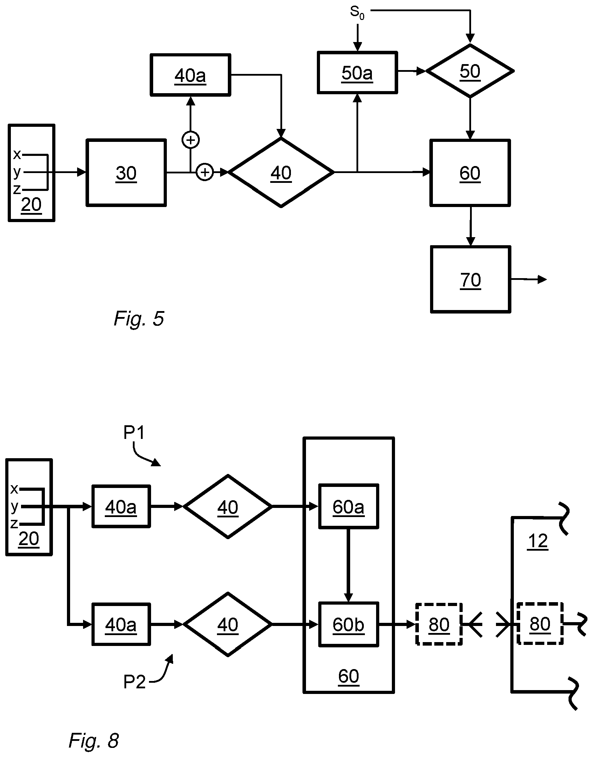

Anhand des Ablaufdiagramms in Fig. 5 wird ein erstes Ausführungsbeispiel des erfindungsgemäßen Verfahrens im Detail beschrieben. Zu Beginn wird die Sondenvorrichtung 1 in den Magen-Darmtrakt 3 eines Nutztiers 2 eingebracht, im dargestellten Ausführungsbeispiel in den Netzmagen einer Kuh. Zu diesem Zweck ist die Sondenvorrichtung 1 mit einer hohen Dichte und angepassten Außenmaßen ausgeführt, so dass sie nach Zuführung im Netzmagen verbleibt, bzw. nach einer kurzen Zeit am Beginn im Pansen in den Netzmagen kommt und dort verbleibt.

20: Mittels eines dreiachsigen Beschleunigungssensors für die drei Achsen - x-Achse, y-Achse und z-Achse - eines kartesischen Koordinatensystems werden als erster physikalischer Parameter rohe Beschleunigungsdaten innerhalb des Magen-Darmtrakts 3 der Kuh 2 ermittelt. Es liegen also Beschleunigungswerte für die x-, y- und z-Achse vor.

Die Ermittlung kann beispielsweise mit einer Samplingrate von 100 Hz erfolgen, wobei das Signal z.B. mit 1 Bit = 15 mg oder besser aufgelöst wird. Die Beschleunigung wird in der vorliegenden Offenbarung als Vielfaches oder Teil des Mittelwerts der Erdbeschleunigung angegeben, die mit dem Buchstaben "*g*" bezeichnet wird und die gerundet ungefähr 9,81 m/s⁻² beträgt.

Die Messung durch den Beschleunigungssensor erfolgt dabei entweder kontinuierlich oder ereignisgesteuert, wobei ein Schwellwert - insbesondere für die Beschleunigung - für die Aktivierung bzw. den Messbeginn vordefiniert sein kann. Hier handelt es sich also um einen Messbeginns-Schwellwert. Die Beschleunigungsdaten werden an die Sondensteuereinheit 6 übermittelt, in der nachfolgend die Messwerte für den ersten physikalischen Parameter in ein Motilitätssignal als erste Zustandsgröße für den Organismus des Nutztiers umgewandelt werden.

30: In einem Pre-Processing werden Absolutwerte (nachfolgend als "abs" bezeichnet) der Beschleunigungen der drei Achsen gebildet. Die Absolutwerte werden verwendet, da auf den einzelnen Achsen positive und negative Beschleunigungswerte auftreten können. Dieser Schritt ist optional - je nachdem, welches Vorgehen bei den nachfolgenden Schritten gewählt wird kann das Bilden der Absolutwerte auch unterbleiben.

40: Die gebildeten Absolutwerte der Beschleunigungen werden summiert. Es gilt:
Beschleunigungssumme = abs(x-Achse) + abs(y-Achse) + abs(z-Achse).

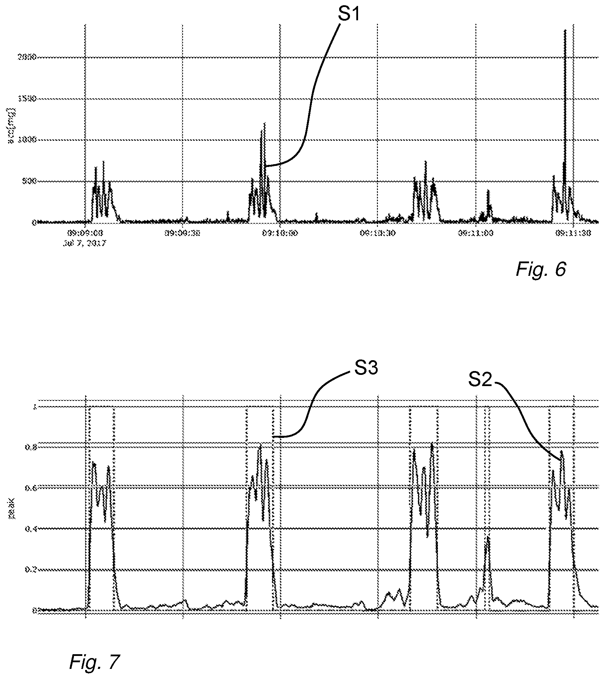

Im Messergebnisse-Diagramm in Fig. 6 ist das resultierende Summensignal S1 für die Messwerte des ersten physikalischen Parameters über einer Zeitachse dargestellt. Dabei ist beispielhaft ein Zeitraum am 7. Juli 2017 von 09:09 Uhr bis 09:11 Uhr ausgewählt. Auf der y-Achse ist die Beschleunigung ("*acc*") in der Einheit mg dargestellt.

In dieser Pre-Processingphase wird durch die Verwendung von Echtzeitinformation eines Taktgebers 53 bzw. einer RTC aus den Summenwerten der Beschleunigung ein äquidistantes diskretes Signal generiert. Der Taktgeber 53 kann dabei z.B. auch relative Zeitinformation ausgeben wie Zeit bzw. Anzahl von Zeiteinheiten seit der letzten Übertragung (erfolgt nach Durchführung der in Fig. 5 dargestellten Abläufe bzw. im zuvor beschriebenen Schritt c)) oder seit Aktivieren der Sondeneinheit 1. Mit anderen Worten kann zum Durchführen der Schritte des beschriebenen Verfahrens auch relative Zeitinformation berücksichtigt werden, die durch einen Taktgeber 53 vorgegeben wird. Dieser Taktgeber kann dabei in der Sondenvorrichtung 1 separat oder als Teil der Sondensteuereinheit 6 vorgesehen sein.

40a: Da das Beschleunigungssignal nicht nur die Motilität, sondern auch andere Beschleunigungen - Bewegung des Nutztieres, Erdbeschleunigung, die oben beschriebenen Einflüsse durch Nahrungsaufnahme - enthält, folgt eine Filterung, um die Detektierbarkeit der Motilität zu verbessern. Dabei können verschiedene Filterverfahren zum Einsatz kommen, beispielsweise Convolution-Filter (Berechnung eines Mittelwerts aus dem Beschleunigungswert eines Datenpunktes und einer bestimmten Anzahl seiner Nachbar-Datenpunkte, Zuweisung dieses Mittelwerts auf diesen Datenpunkt), *"Moving-Average"-*Filter (für ein eine bestimmte Anzahl an Datenpunkte enthaltendes Fenster wird ein Mittelwert berechnet, dieses Fenster wird dabei überlappend bzw. iterativ verschoben, d.h. der erste Datenpunkt aus dem betrachteten Fenster/Ausschnitt wird gestrichen, der erste Datenpunkt nach dem Fenster/Ausschnitt hinzugenommen und ein neuer Mittelwert berechnet. Für die Berechnung des Mittelwerts können die im Fenster vorkommenden Datenpunkte anschließend beliebig gewichtet werden) oder diskrete Tiefpassfilter, die Werte unterhalb einer gewissen Grenzschwelle (hier also eines Beschleunigungswerts) praktisch ungeschwächt passieren lassen. Die Erdbeschleunigung kann beispielsweise hardwareseitig, also direkt am hier verwendeten dreiachsigen Beschleunigungssensor herausgefiltert werden.

Im dargestellten Beispiel kommt ein diskreter Tiefpassfilter zum Einsatz, die Beschleunigungssumme gemäß 40 ist also tiefpassgefiltert. In Fig. 7 ist das tiefpassgefilterte Summensignal S2 des Summensignals S1 aus Fig. 6 dargestellt.

In einer hier nicht dargestellten Variante kann die Filterung auch direkt auf die in Schritt 20 ermittelten Beschleunigungswerte der x-, y- und z-Achse angewandt werden, bevor die Bildung der Beschleunigungssumme erfolgt.

50: Zur Umwandlung der Messwerte des ersten physikalischen Parameters in eine erste Zustandsgröße des Organismus des Nutztieres 2 wird ein Parameter-Schwellwert benötigt. Dabei kann entweder ein fixer Parameter-Schwellwert S₀ herangezogen werden, der statisch ist und über die Konfigurierung bzw. Konfiguration der Sondenvorrichtung 1 bzw. über deren Sendeeinrichtung 8 einstellbar ist. In einer Variante kann ein adaptiver Parameter-Schwellwert verwendet werden, der aus besagtem fixen Parameter-Schwellwert S₀ besteht, der durch einen dynamischen Schwellwert ergänzt wird, der abhängig ist von der jeweiligen Aktivität des Nutztieres über einen gewissen Zeitraum. Dafür kann aus dem tiefpassgefilterten Summensignal gemäß 40 und 40a ein gleitender Mittelwert über einen gewissen Zeitraum ermittelt werden, was in 50a dargestellt ist. Dieser dynamische Schwellwert kann entweder in der Sondensteuereinheit 6 ermittelt werden oder in der Auswerteeinheit 12, die dann den Wert über einen passenden Übertragungsweg einspielt. In einer Variante kann auch der fixe Parameter-Schwellwert S₀ = 0 gesetzt und der Schwellwert rein durch den dynamischen Schwellwert gebildet werden.

60: In einer Peak-Detection werden die Messwerte des ersten physikalischen Parameters umgewandelt in die erste Zustandsgröße. Als Eingangsgrößen dienen dabei das tiefpassgefilterte Summensignal aus 40 bzw. 40a und der Parameter-Schwellwert aus 50 bzw. 50a.

Wenn ein Datenpunkt des Summensignals größer ist als der Parameter-Schwellwert, wird dem Datenpunkt eine binäre "*1*" zugewiesen, allen anderen Datenpunkten wird eine *"0"* zugewiesen. Datenpunkten, die identisch zum Parameter-Schwellwert sind, kann je nach Konfigurierung bzw. Konfiguration der Sondenvorrichtung 1 eine "*1*"oder eine *"0"* zugewiesen werden.

Die erste Zustandsgröße - im vorliegenden Ausführungsbeispiel die Motilität-ergibt sich damit als binäres Rechtecksignal S3, dessen Verlauf durch die punktierte Linie in Fig. 7 dargestellt ist. Datenpunkte mit *"1"* entsprechen einer Kontraktion des Magen-Darmtraktes, bei *"0"* liegen keine Magenbewegungen vor. Insbesondere wird die Kontraktion des Netzmagens (*"Reticulum"* oder auch Haube genannt) ermittelt, wenn die Sondenvorrichtung 1 dort angeordnet ist, die Kontraktionen der anderen Bereiche des Magen-Darmtraktes 3 (insbesondere dorsaler Pansensack, Vorhof, ventraler Pansensack) werden nicht gemessen.

In der Peak-Detection wird damit das tiefpassgefilterte Summensignal der absoluten Beschleunigungswerte in ein binäres Rechtecksignal umgewandelt, das der Motilität als erster Zustandsgröße des Organismus des Nutztieres entspricht. Dieses Rechtecksignal als Abfolge von *"0"* und *"1"* ist mit einer geringeren Datenübertragungsrate übermittelbar als das Summensignal der Beschleunigungen. Dadurch kann der Stromverbrauch beim Übertragen der Messwerte der ersten Zustandsgröße an die Auswerteeinheit 12 deutlich reduziert werden. In einer hier nicht weiter diskutieren Variante der Erfindung können aber auch die Dauer der Kontraktionen der Motilität und/oder deren Periodizität mittels entsprechender Auswertung des Rechtecksignals als erste Zustandsgröße herangezogen werden.

70: Um die zu übertragende Datenmenge weiter zu reduzieren wird das Rechtecksignal in einem Postprocessing *"downsampled"* bzw. heruntergetaktet.

Bei der beschriebenen Samplingrate des Beschleunigungssensors von 100 Hz ergibt sich nach der Peak-Detection 60 ein Rechtecksignal mit ebenfalls 100 Hz.

Im Postprocessing wird darauf eine Heruntertaktung angewandt, z.B. um den Faktor 50, so dass nur ein 2Hz-Rechtecksignal an die Auswerteeinheit 12 übermittelt werden muss. Dabei werden beim angenommenen Taktungsfaktor 50 jeweils 50 Datenpunkte des Rechtecksignals aus der Peak-Detection 60 gesammelt - wenn mehr als die Hälfte dieser Datenpunkte *"1"* ist, wird als Gesamt-Output *"1"* angenommen, bei *"0"* dementsprechend *"0"*. Der dominierende binäre Wert der ausgewählten Menge an Datenpunkten bestimmt damit dessen Gesamtwert.

Das besagte Postprocessing bzw. Heruntertakten kann in nicht dargestellten Ausführungsbeispielen auch schon zu einem früheren Zeitpunkt des beschriebenen Verfahrens erfolgen, beispielsweise nach Ermittlung der Messwerte in Schritt 20 oder nach der Filterung in Schritt 40a.

Eine weitere Reduzierung der zu übertragenden Datenmenge lässt sich erreichen durch Komprimieren der Datenmenge, z.B. durch Lauflängencodierung *("runlength encoding"* bzw. *"RLE"*; es werden nur diejenigen Stellen markiert, an denen sich der binäre Wert ändert) oder Übermitteln von Informationen nur derjenigen Bereiche des Rechtecksignals, in denen der binäre Wert *"1"* beträgt.

Der in Fig. 5 aus dem Block 70 weisende Pfeil bedeutet, dass das Ergebnis der Peak-Detection 60 bzw. des Post-Processing 70 an die Auswerteeinheit 12 übertragen wird, wobei zwischenzeitlich eine Speicherung in einem Speicherelement 7 der Sondenvorrichtung 1 stattfinden kann, um die Messwerte der ersten Zustandsgröße gebündelt zu übertragen.

Die resultierenden binären Werte werden also zuerst in einem Zwischenspeicher auf RAM gespeichert, nach Ablauf eines Messintervalls wird der temporäre Zwischenspeicher auf einen EEPROM als Nur-Lese-Speicher übertragen. Dieser Schritt ist notwendig, um eine ausreichend große Speichermöglichkeit für die Messwerte der ersten Zustandsgröße sicherzustellen, bevor die nächste Übertragung an die Auswerteeinheit 12 erfolgt. Andernfalls kann es zu Datenlücken in den Messwerten der ersten Zustandsgröße kommen.

Nach dem Übermitteln des binären Rechtecksignals der ersten Zustandsgröße an die Auswerteeinheit 12 wird die erste Zustandsgröße auf der Auswerteeinheit in die zweite Zustandsgröße umgewandelt.

Ausgehend von der Motilität als erster Zustandsgröße kann es sich bei der zweiten Zustandsgröße um die Pulsweite und damit Dauer der Kontraktionen und/oder deren Periodizität - also die Zeit, die zwischen zwei aufeinanderfolgenden Kontraktionsphasen vergeht - und/oder deren Frequenz und damit die Rumination bzw. Wiederkäuzeiten handeln.

Die Umwandlung der Motilität als erster Zustandsgröße in die Rumination als zweite Zustandsgröße erfolgt beispielsweise durch Identifikation von Bereichen des binären Rechtecksignals, in denen die Pulsweite der Motilität zuerst zunimmt und dann konstant bleibt, bis sie sich wieder ändert. Ein derartiger Bereich wird als Wiederkäuaktivität identifiziert. Diese Identifizierung erfolgt bevorzugt unter Anwendung geeigneter Algorithmen, beispielsweise von Machine-Learning Algorithms. Der Rechenaufwand für derartige Algorithmen ist vergleichsweise hoch, daher ist es günstig, die Ermittlung der zweiten Zustandsgröße aus der ersten Zustandsgröße in der Auswerteeinheit 12 durchzuführen, während die relativ ressourcensparende Umwandlung des ersten physikalischen Parameters in die erste Zustandsgröße in der Sondensteuereinheit 6 durchgeführt wird.

Dadurch ergibt sich insbesondere auch der Vorteil, dass der für die Umwandlung der ersten in die zweite Zustandsgröße verwendete Algorithmus ohne großen Aufwand geändert bzw. angepasst werden kann - würde die Umwandlung in der Sondenvorrichtung 1 erfolgen, müsste die Änderung der Algorithmen über Updates erfolgen, die eine Beschränkung der für die Sondenvorrichtungen 1 verfügbaren Übertragungskapazität bewirken, was für die bestimmungsgemäße Verwendung der Sondenvorrichtungen 1 im Produktionsbetrieb nachteilig ist.

Fig. 8 zeigt ein zweites Ausführungsbeispiel des erfindungsgemäßen Verfahrens, an dessen Beginn so wie beim ersten Ausführungsbeispiel gemäß Fig. 5 das Einbringen einer Sondenvorrichtung 1 in den Magen-Darmtrakt 3 eines Nutztiers 2 steht.

20: Es werden wieder Beschleunigungswerte für eine x-, y- und z-Achse eines kartesischen Koordinatensystems mittels eines Beschleunigungssensors ermittelt.

Die darauffolgenden Schritte werden in zwei parallelen Pfaden durchgeführt. Der in Fig. 5 dargestellte Schritt 30 ist in Fig. 8 nicht dargestellt, da er optional ist und nicht zwingend durchgeführt werden muss. Beim Ermitteln der Motilität als erster Zustandsgröße wird in dem in Fig. 8 oberen Pfad P1 aus den Beschleunigungswerten eine Positionserkennung einer Motilitätskontraktion durchgeführt, also der Zeitpunkt, zu dem die Kontraktion stattfindet; im unteren Pfad P2 in Fig. 8 wird eine Kontraktionsbreite ermittelt, also die Dauer der Kontraktion, insbesondere auch unter Verwendung der Ergebnisse des oberen Pfades P1.

40a: In diesem Filterschritt erfolgt für beide Pfade P1, P2 eine achsenselektive Filterung wie beim ersten Ausführungsbeispiel beschrieben. "*Achsenselektiv"* bedeutet hier, dass die Beschleunigungswerte der x-, y- und z-Achse separat gefiltert werden, z.B. durch Anwenden eines Bandpassfilters. Dadurch lassen sich Rauschen und Störungen besonders gut entfernen, z.B. Bewegungsaktivitäten des Nutztiers, die die nachfolgenden Abläufe des erfindungsgemäßen Verfahrens nachteilig beeinflussen könnten.

40: Die gefilterten Signale der x-, y- und z-Achse werden in einem Aggregationsschritt zu einem Summensignal kombiniert, das für die nachfolgenden Schritte herangezogen wird. Dabei sind verschiedene Abläufe möglich. Im Zusammenhang mit Fig. 5 wurde bereits das folgende Vorgehen beschrieben:
Beschleunigungssumme = abs(x-Achse) + abs(y-Achse) + abs(z-Achse).

Es können aber auch folgende Varianten zur Anwendung kommen:
Beschleunigungssumme = x-Achse^2 + y-Achse^2 + z-Achse^2; oder
Beschleunigungssumme = sqrt(x-Achse^2 + y-Achse^2 + z-Achse^2).

Die Abkürzung "sqrt" steht dabei für *"square root"*, also die Wurzel; das Symbol "^2" bezeichnet nach üblicher Art die Quadratfunktion. Diese Varianten können bei beiden Pfaden P1, P2 zum Einsatz kommen. Insbesondere beim unteren Pfad P2 zur Ermittlung der Kontraktionsbreite können zusätzlich Varianten verwendet werden, wie z. B.:
Beschleunigungssumme = arctan (y-Achse, z-Achse), wobei hier y-Achse und z-Achse ausgewählt werden, da die Position des Beschleunigungssensors in der Sondeneinheit 1 und die Tatsache, dass die Sondeneinheit 1 im Wesentlichen zylinderförmig ist, zu berücksichtigen sind. Unter Verwendung von r = sqrt(x-Achse^2 + y-Achse^2 + z-Achse^2) kann auch folgende Lösung verwendet werden:
Beschleunigungssumme = arctan (r, x-Achse).

Grundsätzlich ist zu sagen, dass die Reihenfolge von Filterschritt und Aggregationsschritt auch anders gewählt werden kann, so dass die Aggregation vor der Filterung erfolgt oder ein erster Filterschritt vor der Aggregation erfolgt und ein zweiter an die Aggregation angeschlossen wird. Insbesondere können die beiden Pfade P1, P2 insbesondere auch hinsichtlich der Anzahl und Reihenfolge dieser Schritte unterschiedlich ausgeführt werden. Die Positionierung der Blöcke 40a und 40 in Fig. 8 ist also nur eine von mehreren Möglichkeiten.

60: Das Umwandeln der Messwerte des ersten physikalischen Parameters erfolgt im dargestellten Ausführungsbeispiel gemäß Fig. 8 unterschiedlich zur Ausführung in Fig. 5. Hier werden die Pfade P1, P2 wieder zusammengeführt.

60a: Im oberen Pfad P1 zur Positionsermittlung werden durch Anwendung eines Peak-Detection-Algorithmus die zeitlichen Positionen der Motilitäts-Kontraktionen ermittelt. Dabei werden als Input folgende Parameter herangezogen, die entweder in der Sondeneinheit 1 hinterlegt, dynamisch ermittelt oder über die Auswerteeinheit 12 eingespielt werden:
- Schwellwert, insbesondere adaptiver Schwellwert, der ähnlich dem Parameter-Schwellwertn aus Fig. 5 der wie in den dort dargestellten Schritten 50, 50a (aus Gründen der Übersichtlichkeit sind diese Schritte in Fig. 8 nicht dargestellt) über einen gleitenden Mittelwert oder *"rolling root mean square"* ermittelt wird;
- Minimaler Kontraktionsabstand, bezeichnet den kleinsten zulässigen zeitlichen Abstand zwischen zwei Kontraktionen des Netzmagens;
- Beschleunigungssumme; das gefilterte und aggregierte Rohsignal des Beschleunigungssensors wie oben beschrieben.

Die zeitlichen Positionen der Kontraktionen werden nun so ermittelt, dass allen Werten der Beschleunigungssumme, die größer sind als der Schwellwert, der Wert *"1"* zugeordnet wird, für die übrigen Werte gilt der Wert *"0"*. Die Werte der Beschleunigungssumme, denen der Wert *"1"* zugeordnet wird, werden nachfolgend als *"peaks"* bezeichnet.

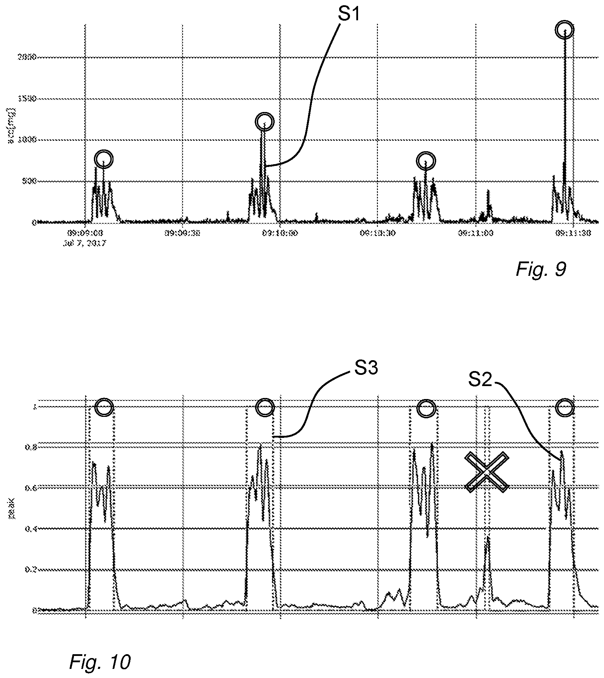

Die *"peaks"* werden nun noch dahingehend selektiert, ob sie zueinander näher liegen als der oben definierte minimale Kontraktionsabstand. Bei zwei *"peaks",* deren Abstand zueinander unter dem minimalen Kontraktionsabstand liegt, wird derjenige *"peak"* eliminiert, dessen Beschleunigungssumme geringer ist. Der größere "*peak*" bleibt erhalten. Es werden solange *"peaks"* eliminiert bis der zeitliche Abstand zwischen den verbleibenden *"peaks"* größer ist als der definierte minimale Kontraktionsabstand. Fig. 9 zeigt nun eine Anwendung dieses Verfahrens auf das Summensignal S1 aus Fig. 6, wobei die identifizierten und verbleibenden "*peaks*" mit Kreisen mit Doppellinie gekennzeichnet sind.

60b: Im unteren Pfad P2 zur Ermittlung der Dauer bzw. Breite der Kontraktionen wird auf die Beschleunigungssumme neben einem Schwellwert zur Identifizierung von *"peaks"* ein Parameter angewandt, der einen minimalen Abstand zwischen "*peaks*" (minimaler Peakabstand) definiert. Dadurch werden *"peaks",* die näher zueinander sind als der minimale Peakabstand, vereint zu einem gemeinsamen *"peak".* Wie in Fig. 10 erkennbar ist ergibt sich dadurch eine Abfolge von Bereichen mit dem Wert *"0"*- kein *"peak"* - und von Bereichen mit dem Wert "*1*"-vereinigte *"peaks",* die vor Anwendung des Parameters näher zueinander lagen als der definierte minimale Peakabstand. Darauf wird nun noch das Ergebnis von 60a im oberen Pfad P1 angewandt: Diejenigen vereinigten *"peaks",* die im Schritt 60a nicht als Position einer Kontraktion identifiziert wurden, werden eliminiert-ein "*peak"* in Fig. 10 ist daher gestrichen.

Als Ergebnis des Schritts 60 mit den Unterschritten 60a und 60b in Fig. 8 ergibt sich also wieder ein Rechtecksignal, die Pfade P1, P2 sind wieder zusammengeführt.

Auch im Verfahren gemäß Fig. 8 kann ein Post-Processing zum Einsatz kommen, wie es im Zusammenhang mit Fig. 5 beschrieben wurde. Allerdings kann es an einer oder mehreren verschiedenen Stellen vorgenommen werden und ist daher aus Gründen der Übersichtlichkeit nicht eingezeichnet.

Das in Fig. 8 dargestellte zweite Ausführungsbeispiel hat den Vorteil, dass das Motilitätssignal präziser ermittelt werden kann, was das weitere Verfahren - insbesondere das Ermitteln der zweiten Zustandsgröße, z.B. der Periodizität, also der Zeit zwischen Kontraktionen, erleichtert. Außerdem können Signale, die nicht als Motilitätssignal zu betrachten sind, eliminiert werden.

Im Anschluss an das Umwandeln der Messwerte des ersten physikalischen Parameters in die erste Zustandsgröße kann noch ein Validierungsschritt 80 vorgenommen werden. Der Validierungsschritt 80 kann entweder auf der Sondeneinheit 1 vor Übermittlung an die Auswerteeinheit 12, also als Unterschritt von Schritt b) bzw. vor Durchführung des Schritts c) des erfindungsgemäßen Verfahrens, vorgenommen werden, oder erst auf der Auswerteeinheit 12 als Teil von Schritt d) des erfindungsgemäßen Verfahrens. Aus diesem Grund ist der Validierungsschritt 80 in Fig. 8 als strichlierter Kasten sowohl anschließend an Schritt 60 als auch als zugehörig zur Auswerteeinheit 12 dargestellt. Grundsätzlich wäre es auch möglich, dass eine derartige Validierung zwei Mal stattfindet, dass also beide strichlierte Schritte 80 ausgeführt werden.

Ein Validierungsschritt 80 ist insbesondere deshalb nötig, weil aufgrund des komplexen Umfelds der Datennahme - Bewegung des Nutztieres, Einfluss von Umweltbedingungen, unerwartete Ereignisse - fehlerhafte Messdaten nicht ausgeschlossen werden können. Bei Ermittlung der Motilität aus Beschleunigungswerten kann es aufgrund von hoher Bewegungsaktivität des Nutztiers oder wenn sich die Sondeneinheit 1 aus dem Netzmagen herausbewegt zu Fehlmessungen kommen.

Im dargestellten Ausführungsbeispiel kann der Validierungschritt 80 beispielsweise so vorgenommen werden, dass die erste Zustandsgröße in Form des Motilitätssignals validiert wird. Bei dieser Motilitäts-Validierung wird überprüft, ob die Pulsbreiten - im Wesentlichen also die Dauer der Kontraktionen, bei denen der Wert des Rechtecksignals *"1"* beträgt - und/oder die im Schritt 60 angewandten Schwellwerte, die ja teilweise adaptiv verändert werden, plausibel sind. Wenn die Pulsbreite bekanntermaßen zwischen 5 und 15 Sekunden liegen muss kann eine ermittelte Pulsbreite von mehr als 30 Sekunden als fehlerhaft erkannt werden. Je nachdem, welcher Wert als erste Zustandsgröße herangezogen wird kann im Validierungsschritt 80 auch geprüft werden, ob eine resultierende Periodizität plausibel ist und/oder einwirkende Beschleunigungen während der Kontraktion plausibel sind. Entsprechende Plausibilitätswerte sind auf der Sondeneinheit 1 und/oder der Auswerteeinheit 12 hinterlegt bzw. werden zwischen diesen ausgetauscht, insbesondere dahingehend, dass von der Auswerteeinheit 12 entsprechende Werte an die Sondeneinheit 1 übermittelt werden.

Günstigerweise wird dieser Validierungsschritt 80 auf der Sondeneinheit 1 durchgeführt, weil sich dadurch verhindern lässt, dass das Übertragen von fehlerhaften Daten auf Kosten der Energie und Bandbreite vorgenommen wird. Daten, die den Validierungsschritt 80 nicht bestehen, werden nur als Fehlersignal übermittelt. Auf der Auswerteeinheit 12 werden dann derartige Daten nicht berücksichtigt.

Natürlich kann der Validierungsschritt 80 auch erst auf der Auswerteeinheit 12 erfolgen, wo dann nur die plausiblen Daten weiter verarbeitet werden.

Bei den in Ausführungsbeispielen beschriebenen computergestützten Verfahren zur Ermittlung zumindest einer Zustandsgröße des Organismus zumindest eines Nutztieres 2 wird also auf einer Sondenvorrichtung 1 ein erster physikalischer Parameter ermittelt, beispielsweise Beschleunigungswerte mithilfe eines dreiachsigen Beschleunigungssensors, und in einer Sondensteuereinheit 6 in die Motilität als erste Zustandsgröße des Organismus des Nutztieres 2 in Form eines binären Rechtecksignals umgewandelt, aus dem Informationen über den Organismus des Nutztieres nach wie vor ermittelbar sind. Dieser Vorgang benötigt nur geringe Rechenkapazitäten und bewirkt eine Reduzierung der Datenmenge. Außerdem wird das Risiko von Fehlern im Source-Code durch die vergleichsweise einfache Programmierung reduziert.

Diese erste Zustandsgröße wird über ein geeignetes Übertragungsverfahren, insbesondere ein drahtloses Verfahren, an eine Auswerteeinheit 12 übertragen. Die Menge an zu übertragenden Daten ist vergleichsweise gering, was die Übertragungszeit, den damit verbundenen Stromverbrauch auf der Sondenvorrichtung 1 und die benötigte Bandbreite reduziert. In der Auswerteeinheit 12 wird die erste Zustandsgröße in vergleichsweise aufwändigeren Verfahren in eine zweite Zustandsgröße, die Rumination bzw. Wiederkäuaktivität, umgewandelt. Diese Umwandlung kann ohne Probleme ressourcenintensiv ausgelegt werden, weil die Auswerteeinheit 12 - üblicherweise ein Computer bzw. Server - über ausreichend Rechenkapazität verfügt und als stationäre Einheit einfach mit Energie versorgt werden kann.

Damit kann eine langfristige ordnungsgemäße Verwendung der Sondenvorrichtung 1 bei bestmöglichem Informationaustausch mit der Auswerteeinheit 12 sichergestellt werden.

## Patentansprüche

1. Verfahren zur Ermittlung zumindest einer Zustandsgröße des Organismus zumindest eines Nutztieres (2), wobei zumindest eine Sondenvorrichtung (1) zur Messung zumindest eines physikalischen Parameters im Magen-Darmtrakt (3) des Nutztieres (2) angeordnet ist, und zumindest eine Auswerteeinheit (12) außerhalb des Magen-Darmtraktes (3) des Nutztieres (2) angeordnet ist, **gekennzeichnet durch** die folgenden Schritte:
a1) Ermitteln der Beschleunigung der Sondenvorrichtung (1) in alle drei Raumrichtungen eines kartesischen Koordinatensystems;
a2) Ermitteln der Beschleunigung gemäß Schritt a1) als ersten physikalischen Parameter durch die Sondenvorrichtung (1) durch Durchführen zumindest eines der folgenden Schritte: Summieren von Absolutwerten der in Schritt a1) ermittelten Beschleunigungswerte, oder Summieren der Quadrate der in Schritt a1) ermittelten Beschleunigungswerte, oder Wurzel der Summe der Quadrate der in Schritt a1) ermittelten Beschleunigungswerte, oder arctan der in Schritt a1) ermittelten Beschleunigungswerte für y-Achse und z-Achse des kartesischen Koordinatensystems, oder arctan der Wurzel der Summe der Quadrate der in Schritt a1) ermittelten Beschleunigungswerte und der x-Achse des kartesischen Koordinatensystems;
b) Umwandeln des ersten physikalischen Parameters in die Motilität als eine erste Zustandsgröße des Organismus des Nutztieres (2) in einer Sondensteuereinheit (6) der Sondenvorrichtung (1), wobei Messwerten des ersten physikalischen Parameters, die größer, insbesondere größer oder gleich einem Schwellwert sind, der Wert einer binären "1" zugeordnet wird und Messwerten des ersten physikalischen Parameters, die kleiner sind als ein Schwellwert der Wert "0" zugeordnet wird, wobei der Schwellwert entweder vordefiniert auf der Sondenvorrichtung (1) hinterlegt wird oder von der Auswerteeinheit (12) anlassbezogen an die Sondenvorrichtung (1) übermittelt wird oder aus einem Basis-Schwellwert und einem in Abhängigkeit vom Verhalten des ersten physikalischen Parameters anpassbaren, zum Basis-Schwellwert addierten oder vom Basis-Schwellwert subtrahierten Dynamik-Wert besteht;
c) Übermitteln der ersten Zustandsgröße als binäres Rechtecksignal von der Sondenvorrichtung (1) an die Auswerteeinheit (12);
d) Umwandeln der ersten Zustandsgröße in eine zweite Zustandsgröße in der Auswerteeinheit (12),
wobei die Ermittlung des ersten physikalischen Parameters in Schritt a) auf zumindest eine der folgenden Arten erfolgt: kontinuierlich; in regelmäßigen Abständen; kontinuierlich, sobald ein von der Sondenvorrichtung (1) gemessener physikalischer Parameter, insbesondere der erste physikalische Parameter, einen Schwellwert überschritten hat; in regelmäßigen Abständen, sobald ein von der Sondenvorrichtung (1) gemessener physikalischer Parameter, insbesondere der erste physikalische Parameter, einen Schwellwert überschritten hat; kontinuierlich oder in regelmäßigen Abständen für einen vordefinierten Zeitraum, sobald ein von der Sondenvorrichtung (1) gemessener physikalischer Parameter, insbesondere der erste physikalische Parameter, einen Schwellwert überschreitet.

2. Verfahren nach Anspruch 1, **dadurch gekennzeichnet, dass** es sich bei der ersten Zustandsgröße um eine Zustandsgröße handelt, die in Schritt c) mit geringerer Datenübertragungsrate übermittelbar ist als der erste physikalische Parameter und/oder die zweite Zustandsgröße.

3. Verfahren nach Anspruch 1 oder 2, **dadurch gekennzeichnet, dass** der erste physikalische Parameter mit geringerem Rechenaufwand in die erste Zustandsgröße umwandelbar ist als der erste physikalische Parameter in die zweite Zustandsgröße und/oder die erste Zustandsgröße in die zweite Zustandsgröße.

4. Verfahren nach einem der Ansprüche 1 bis 3; **dadurch gekennzeichnet, dass** es sich bei der ersten Zustandsgröße um die Dauer und/oder die Periodizität und/oder die Frequenz der Motilität handelt.

5. Verfahren nach einem der Ansprüche 1 bis 4, **dadurch gekennzeichnet, dass** in Schritt b) die erste Zustandsgröße mit einem Echtzeitsignal eines in der Sondenvorrichtung (1) vorgesehenen Taktgebers (53) und/oder einem Temperatursignal eines in der Sondenvorrichtung (1) vorgesehenen Temperatursensors (52) verifiziert wird.

6. Verfahren nach einem der Ansprüche 1 bis 5, **dadurch gekennzeichnet, dass** es sich bei der zweiten Zustandsgröße in Schritt d) um eine der folgenden Zustandsgrößen des Organismus des Nutztieres (2) handelt: Rumination, Herzschlag, Fresszeit.

7. Verfahren nach einem der Ansprüche 1 bis 6, **dadurch gekennzeichnet, dass** unmittelbar vor Schritt c) in einer Sondensteuereinheit (6) der Sondenvorrichtung (1) und/oder vor Durchführung des Schritts d) in der Auswerteeinheit (12) ein Validierungsschritt (80) durchgeführt wird, um die Plausibilität der Werte der ersten Zustandsgröße des Organismus des Nutztieres (2) zu prüfen.

8. Sondenvorrichtung (1) zur Messung zumindest einer Zustandsgröße des Organismus eines Nutztieres (2), wobei die Sondenvorrichtung (1) im Magen-Darmtrakt (3) des Nutztieres (2) anordenbar ist und zumindest folgende, in einem Gehäuse (4) angeordnete Komponenten aufweist:
- zumindest ein Sensorelement (51, 52) zur Messung zumindest eines physikalischen Parameters im Magen-Darmtrakt (3) des Nutztieres (2),
- zumindest eine Sendeeinrichtung (8), vorzugsweise mit zumindest einer Antenne (9), zum drahtlosen Übertragen und Empfangen von Information, und
- zumindest eine Sondensteuereinheit (6) die zur Durchführung der Schritte a) bis c) eines Verfahrens nach einem der Ansprüche 1 bis 7 eingerichtet ist.

9. Sondenvorrichtung (1) nach Anspruch 8, **dadurch gekennzeichnet, dass** zumindest eines der folgenden Sensorelemente vorgesehen ist: Beschleunigungssensor, Beschleunigungssensor zur Messung der Beschleunigung in alle drei Raumrichtungen eines kartesischen Koordinatensystems, Temperatursensor, ph-Sensor, Taktgeber, Echtzeituhr, Kameraelement.

10. Sondenvorrichtung (1) nach Anspruch 8 oder 9, **dadurch gekennzeichnet, dass** zumindest ein mit der Sondensteuereinheit (6) verbundenes Speicherelement (7) vorgesehen ist, insbesondere zumindest ein RAM- und/oder zumindest ein ROM-Speicherelement.

11. System (100) mit zumindest einer Auswerteeinheit (12) und zumindest einer Sondenvorrichtung (1) nach einem der Ansprüche 8 bis 10, wobei mit dem System ein Verfahren nach einem der Ansprüche 1 bis 7 durchführbar ist.

## Claims

1. Method for determining at least one state variable of the organism of at least one farm animal (2), at least one probe device (1) for measuring at least one physical parameter being arranged in the gastrointestinal tract (3) of the farm animal (2), and at least one evaluation unit (12) being arranged outside the gastrointestinal tract (3) of the farm animal (2), **characterized by** the following steps:
a1) Determining the acceleration of the probe device (1) in all three spatial directions of a Cartesian coordinate system;
a2) determining the acceleration according to step a1) as the first physical parameter by the probe device (1) by performing at least one of the following steps: summing absolute values of the acceleration values determined in step a1), or summing the squares of the acceleration values determined in step a1), or root of the sum of the squares of the acceleration values determined in step a1), or arctan of the acceleration values determined in step a1) for y-axis and z-axis of the Cartesian coordinate system, or arctan of the root of the sum of the squares of the acceleration values determined in step a1) and the x-axis of the Cartesian coordinate system;
b) converting the first physical parameter into the motility as a first state variable of the organism of the farm animal (2) in a probe control unit (6) of the probe device (1), measured values of the first physical parameter which are greater than, in particular greater than or equal to, a threshold value being assigned the value of a binary "1" and measured values of the first physical parameter which are less than a threshold value being assigned the value of a binary "1", which are smaller than a threshold value are assigned the value "0", wherein the threshold value is either stored predefined on the probe device (1) or is transmitted by the evaluation unit (12) to the probe device (1) on an event-related basis or consists of a basic threshold value and a dynamic value which can be adapted as a function of the behavior of the first physical parameter and is added to the basic threshold value or subtracted from the basic threshold value;
c) transmitting the first state variable as a binary square-wave signal from the probe device (1) to the evaluation unit (12);
d) converting the first state variable into a second state variable in the evaluation unit (12),
wherein the determination of the first physical parameter in step a) is carried out in at least one of the following ways: continuously; at regular intervals; continuously, as soon as a physical parameter measured by the probe device (1), in particular the first physical parameter, has exceeded a threshold value; at regular intervals, as soon as a physical parameter measured by the probe device (1), in particular the first physical parameter, has exceeded a threshold value; continuously or at regular intervals for a predefined period of time, as soon as a physical parameter measured by the probe device (1), in particular the first physical parameter, exceeds a threshold value.

2. Method according to claim 1, **characterized in that** the first state variable is a state variable which can be transmitted in step c) at a lower data transmission rate than the first physical parameter and/or the second state variable.

3. Method according to claim 1 or 2, **characterized in that** the first physical parameter can be converted into the first state variable with less computational effort than the first physical parameter can be converted into the second state variable and/or the first state variable can be converted into the second state variable.

4. Method according to one of claims 1 to 3; **characterized in that** the first state variable is the duration and/or the periodicity and/or the frequency of the motility.

5. Method according to one of claims 1 to 4, **characterized in that** in step b) the first state variable is verified with a real-time signal of a clock (53) provided in the probe device (1) and/or a temperature signal of a temperature sensor (52) provided in the probe device (1).

6. Method according to one of claims 1 to 5, **characterized in that** the second state variable in step d) is one of the following state variables of the organism of the farm animal (2): Rumination, heartbeat, feeding time.

7. Method according to one of claims 1 to 6, **characterized in that** a validation step (80) is carried out immediately before step c) in a probe control unit (6) of the probe device (1) and/or before step d) is carried out in the evaluation unit (12), in order to check the plausibility of the values of the first state variable of the organism of the farm animal (2).

8. Probe device (1) for measuring at least one state variable of the organism of a farm animal (2), wherein the probe device (1) can be arranged in the gastrointestinal tract (3) of the farm animal (2) and has at least the following components arranged in a housing (4):
- at least one sensor element (51, 52) for measuring at least one physical parameter in the gastrointestinal tract (3) of the farm animal (2),
- at least one transmitting device (8), preferably with at least one antenna (9), for wirelessly transmitting and receiving information, and
- at least one probe control unit (6) which is set up to carry out steps a) to c) of a method according to one of claims 1 to 7.

9. Probe device (1) according to claim 8, **characterized in that** at least one of the following sensor elements is provided: acceleration sensor, acceleration sensor for measuring the acceleration in all three spatial directions of a Cartesian coordinate system, temperature sensor, pH sensor, clock generator, real-time clock, camera element.

10. Probe device (1) according to claim 8 or 9, **characterized in that** at least one memory element (7) connected to the probe control unit (6) is provided, in particular at least one RAM and/or at least one ROM memory element.

11. System (100) with at least one evaluation unit (12) and at least one probe device (1) according to one of claims 8 to 10, wherein a method according to one of claims 1 to 7 can be carried out with the system.

## Revendications

1. Procédé pour déterminer au moins une grandeur d'état de l'organisme d'au moins un animal utile (2), au moins un dispositif de sonde (1) étant disposé dans le tractus gastro-intestinal (3) de l'animal utile (2) pour mesurer au moins un paramètre physique, et au moins une unité d'évaluation (12) étant disposée en dehors du tractus gastro-intestinal (3) de l'animal utile (2), **caractérisé par** les étapes suivantes :
a1) Détermination de l'accélération du dispositif de sonde (1) dans les trois directions spatiales d'un système de coordonnées cartésiennes ;
a2) détermination de l'accélération selon l'étape a1) comme premier paramètre physique par le dispositif de sonde (1) en effectuant au moins l'une des étapes suivantes : additionner les valeurs absolues des valeurs d'accélération déterminées à l'étape a1), ou additionner les carrés des valeurs d'accélération déterminées à l'étape a1), ou racine de la somme des carrés des valeurs d'accélération déterminées à l'étape a1), ou arctan des valeurs d'accélération déterminées à l'étape a1) pour l'axe y et l'axe z du système de coordonnées cartésiennes, ou arctan de la racine de la somme des carrés des valeurs d'accélération déterminées à l'étape a1) et l'axe x du système de coordonnées cartésiennes ;
b) conversion du premier paramètre physique en motilité en tant que première grandeur d'état de l'organisme de l'animal utile (2) dans une unité de commande de sonde (6) du dispositif de sonde (1), la valeur d'un « 1 » binaire étant attribuée à des valeurs de mesure du premier paramètre physique qui sont supérieures, en particulier supérieures ou égales à une valeur seuil, et des valeurs de mesure du premier paramètre physique, qui sont inférieures à une valeur seuil, la valeur « 0 » est attribuée, la valeur seuil étant soit déposée de manière prédéfinie sur le dispositif de sonde (1), soit transmise au dispositif de sonde (1) par l'unité d'évaluation (12) en fonction de la situation, soit constituée d'une valeur seuil de base et d'une valeur dynamique adaptable en fonction du comportement du premier paramètre physique, ajoutée à la valeur seuil de base ou soustraite de la valeur seuil de base ;
c) transmettre la première grandeur d'état sous forme de signal rectangulaire binaire du dispositif de sonde (1) à l'unité d'évaluation (12) ;
d) conversion du premier paramètre d'état en un deuxième paramètre d'état dans l'unité d'évaluation (12),
la détermination du premier paramètre physique à l'étape a) étant effectuée de l'une au moins des manières suivantes : en continu ; à intervalles réguliers ; en continu dès qu'un paramètre physique mesuré par le dispositif de sonde (1), notamment le premier paramètre physique, a dépassé une valeur seuil ; à intervalles réguliers dès qu'un paramètre physique mesuré par le dispositif de sonde (1), notamment le premier paramètre physique, a dépassé une valeur seuil ; en continu ou à intervalles réguliers pendant une période prédéfinie dès qu'un paramètre physique mesuré par le dispositif de sonde (1), notamment le premier paramètre physique, a dépassé une valeur seuil.

2. Procédé selon la revendication 1, **caractérisé en ce que** la première grandeur d'état est une grandeur d'état qui peut être transmise à l'étape c) avec un débit de transmission de données plus faible que le premier paramètre physique et/ou la deuxième grandeur d'état.

3. Procédé selon la revendication 1 ou 2, **caractérisé en ce que** le premier paramètre physique peut être converti en la première grandeur d'état avec un effort de calcul moindre que le premier paramètre physique en la deuxième grandeur d'état et/ou la première grandeur d'état en la deuxième grandeur d'état.

4. Procédé selon l'une des revendications 1 à 3 ; **caractérisé en ce que** la première variable d'état est la durée et/ou la périodicité et/ou la fréquence de la motilité.

5. Procédé selon l'une des revendications 1 à 4, **caractérisé en ce que**, à l'étape b), la première grandeur d'état est vérifiée avec un signal en temps réel d'une horloge (53) prévue dans le dispositif de sonde (1) et/ou un signal de température d'un capteur de température (52) prévu dans le dispositif de sonde (1).

6. Procédé selon l'une des revendications 1 à 5, **caractérisé en ce que** la deuxième grandeur d'état à l'étape d) est l'une des grandeurs d'état suivantes de l'organisme de l'animal d'élevage (2) : Rumination, rythme cardiaque, temps d'ingestion.

7. Procédé selon l'une des revendications 1 à 6, **caractérisé en ce qu'**une étape de validation (80) est effectuée immédiatement avant l'étape c) dans une unité de commande de sonde (6) du dispositif de sonde (1) et/ou avant la réalisation de l'étape d) dans l'unité d'évaluation (12), afin de vérifier la plausibilité des valeurs de la première grandeur d'état de l'organisme de l'animal d'élevage (2).

8. Dispositif de sonde (1) pour mesurer au moins une grandeur d'état de l'organisme d'un animal de rente (2), le dispositif de sonde (1) pouvant être disposé dans le tractus gastro-intestinal (3) de l'animal de rente (2) et présentant au moins les composants suivants, disposés dans un boîtier (4) :
- au moins un élément capteur (51, 52) pour mesurer au moins un paramètre physique dans le tractus gastro-intestinal (3) de l'animal utile (2),
- au moins un dispositif d'émission (8), de préférence avec au moins une antenne (9), pour la transmission et la réception sans fil d'informations, et
- au moins une unité de commande de sonde (6) qui est agencée pour exécuter les étapes a) à c) d'un procédé selon l'une des revendications 1 à 7.

9. Dispositif de sonde (1) selon la revendication 8, **caractérisé en ce qu'**il est prévu au moins l'un des éléments de détection suivants : capteur d'accélération, capteur d'accélération pour mesurer l'accélération dans les trois directions spatiales d'un système de coordonnées cartésiennes, capteur de température, capteur de ph, horloge, horloge en temps réel, élément de caméra.

10. Dispositif de sonde (1) selon la revendication 8 ou 9, **caractérisé en ce qu'**il est prévu au moins un élément de mémoire (7) relié à l'unité de commande de sonde (6), en particulier au moins un élément de mémoire RAM et/ou au moins un élément de mémoire ROM.

11. Système (100) comprenant au moins une unité d'évaluation (12) et au moins un dispositif de sonde (1) selon l'une des revendications 8 à 10, le système permettant de mettre en oeuvre un procédé selon l'une des revendications 1 à 7.
